# EUROPÄISCHE PATENTSCHRIFT

(11) **EP 4 602 905 B1**
(45) Veröffentlichungstag und Bekanntmachung des Hinweises auf die Patenterteilung: **11.11.2026**
(21) Anmeldenummer: 25156144.5
(22) Anmeldetag: 06.02.2025
(51) Int. Cl.: A01C 1/02

(54) **GASMESSSYSTEM ZUR BESTIMMUNG EINES QUALITÄTSPARAMETERS VON SAATGUT**
GAS MEASUREMENT SYSTEM FOR DETERMINING A QUALITY PARAMETER OF SEEDS
SYSTÈME DE MESURE DE GAZ POUR DÉTERMINER UN PARAMÈTRE DE QUALITÉ DE SEMENCES

(30) Priorität: 15.02.2024 DE 102024104274
(43) Veröffentlichungstag der Anmeldung: 20.08.2025
(73) Patentinhaber: Dräger Safety AG & Co. KGaA, 23560 Lübeck (DE)
(72) Erfinder: Mattern-Frühwald, Marie-Isabell, 23558 Lübeck (DE); Sick, Michael, 23558 Lübeck (DE); Dennier, Silja, 23558 Lübeck (DE); Rittemann, Steffen, 23558 Lübeck (DE)

(56) Entgegenhaltungen:
- WO-A2-2006/094748
- CN-A- 108 303 134
- JP-A- 2004 239 832

## Beschreibung

Die vorliegende Erfindung betrifft ein Gasmesssystem zur Bestimmung eines Qualitätsparameters von Saatgut.

Es ist ein Ziel bei dem kommerziellen Einsatz von Saatgut, ein Saatgut mit möglichst hoher Qualität zu verwenden, um eine hohe Ausbeute zu erzielen. Hierdurch besteht die Notwendigkeit entlang verschiedener Stufen der Saatgut-Erzeugung eine Aussage über die Qualität des Saatguts treffen zu können.

In der kommerziellen Saatgut-Herstellung wird die Qualität des Saatguts mittels aufwendiger, überwiegend manueller Verfahren bestimmt. Dabei werden Keimlinge einzeln zu Pflanzen gezogen und über eine Probengröße von mindestens 100 Pflanzen eine Einschätzung über ihre Keimfähigkeit erstellt. Die Einschätzung erfolgt nach überwiegend subjektiven, optischen Kriterien.

Weitere Untersuchungsmethoden sind bekannt, wie beispielsweise ein Tetrazolium-Test (Farbtest der vitalen Samen), ein On-farm-Vigour Check mit reaktiven Probenkörpern, bei denen sich die Farbe bei Anwesenheit von Ethanol verändert und aufwendige Röntgenuntersuchungen der Samen.

Es gibt Bestrebungen in der Forschung neue Methoden zu entwickeln. Insofern ist aus der Veröffentlichung Buckley, W. et al., Canola Seed Vigour Ethanol Test, 2003, S. 150-156 eine Methode bekannt, bei der eine Vielzahl von Samen in einem gemeinsamen Gefäß zum Keinem gebracht werden, aus dem mittels einem nicht für diesen Zweck optimierten Gasmessgerät nach einer vorbestimmten Zeit einmalig eine Ethanolprobe entnommen wird. Aus diesen Versuchen ist bekannt, dass eine Menge von während des Keimungsprozesses produziertem Ethanol in direktem Zusammenhang mit der Güte der Samen steht.

Ohne an diese Theorie gebunden zu sein, gehen die Erfinder davon aus, dass während des Keimungsbeginns eines qualitativ guten Samens eine anaerobe Energiegewinnung stattfindet, welche mit einer Spaltung von Glucose zu Laktose und Ethanol verbunden ist. Nach einer Weile stellt der gute Samen dann auf einen aeroben Stoffwechsel um, in welchem Sauerstoff aus der Umgebung in einer nachgelagerten Reaktion verbraucht und kein Ethanol mehr gebildet wird. Bei geschädigten, behandelten oder unreifen Samen wird die anaerobe Energiegewinnung hingegen fortgesetzt, und - da keine Umstellung erfolgt - weiter Ethanol produziert.

Es hat sich jedoch gezeigt, dass diese Methode eine geringe Genauigkeit der Bestimmung der Keimfähigkeit aufweist und ein manuelles Interagieren mit den Samen erfordert.

Aus CN 108 303 134 A ist ein System zum Erfassen von Umgebungsparametern während der Samenkeimung bekannt. Das System umfasst eine Erfassungsvorrichtung, die so eingerichtet ist, dass sie Umgebungsinformationen von Samen in einem begrenzten Raum während der Keimung erfasst, wobei die Umgebungsinformationen unter anderem Sauerstoffkonzentrationsinformationen und Kohlendioxidkonzentrationsinformationen umfassen. Aus den Umgebungsinformationen wird ein "seed vigor" als Indikator einer Saatgutqualität bestimmt.

Es ist daher eine Aufgabe der vorliegenden Erfindung, ein Gasmesssystem zur Bestimmung eines Qualitätsparameters von Saatgut bereitzustellen, mit welchem auf einfache, reproduzierbare Weise eine genaue Aussage über die Qualität einer Saatgutprobe getroffen werden kann.

Diese und weitere Aufgaben werden gelöst durch den Gegenstand der vorliegenden Erfindung gemäß Anspruch 1.

Die abhängigen Ansprüche, die Beschreibung und die Figuren stellen vorteilhafte Ausgestaltungen der vorliegenden Erfindung bereit.

Das erfindungsgemäße Gasmesssystem zur Bestimmung eines Qualitätsparameters von Saatgut weist auf: einen Probenraum zur Aufnahme (vorzugsweise genau) eines Samens des Saatguts, eine Anzahl von elektrochemischen Gassensoren, welche strömungstechnisch mit dem Probenraum verbunden ist, um eine Anzahl von Konzentrationen einer Anzahl von Zielkomponenten eines in dem Probenraum vorhandenen Gases zu bestimmen und mit der Anzahl von Konzentrationen korrespondiere Messignale bereitzustellen, wobei die Bestimmung der Anzahl von Konzentrationen und die Bereitstellung der Messsignale kontinuierlich erfolgt, wobei die Zielkomponenten zumindest Ethanol und Sauerstoff umfassen, und eine Auswerteeinheit, welche eingerichtet ist: die Messsignale automatisch zu empfangen, den Qualitätsparameter aus den Messsignalen zu bestimmen, und den Qualitätsparameter über eine datentechnische Schnittstelle bereitzustellen.

Auf diese Weise kann ein Gasmesssystem bereitgestellt werden, welches eine automatische und objektive Bereitstellung des Qualitätsparameters ermöglicht. Besonders vorteilhaft gegenüber vorbekannten Methoden zur Bestimmung von Qualitätsparametern von Saatgut ist es zum einen, dass eine kontinuierliche Bestimmung der Anzahl von Konzentrationen und Bereitstellung der Messsignale erfolgt, was bei lediglich einmaliger Messung einer Ethanolkonzentration nicht möglich ist. Hierdurch kann ein zeitaufgelöster Verlauf der Konzentration der Anzahl von Zielkomponenten ermittelt werden und so mehr Informationen und bessere Informationen über den Keimungsprozess gewonnen werden und in die Bestimmung des Qualitätsparameters einfließen. Im Rahmen der Erfindung wurde insofern erkannt, dass die Qualität von Saatgut und damit auch der Qualitätsparameter nicht lediglich durch die gesamte während einer Messdauer gebildeten oder verbrauchten Menge der Anzahl von Zielkomponenten indiziert bzw. beeinflusst wird, sondern insbesondere durch den zeitlichen Verlauf der Anzahl von Zielkomponenten.

Zum anderen ist es mit dem erfindungsgemäßen Gasmesssystem möglich, eine Einzelsamenmessung (auch bezeichnet als Einzelkornmessung) oder parallel eine Mehrzahl von jeweiligen Einzelsamenmessungen durchzuführen. Hierdurch ist eine Aussage über die Qualität jedes Samens einer Samenmenge bereitstellbar.

Insgesamt kann somit ein Gasmesssystem bereitgestellt werden, dass eine detaillierte Überwachung der gesamten Keimungsphase eines Samens ermöglicht. Zudem kann auf Ebene einzelner Samen untersucht werden, welche Unterschiede und Gemeinsamkeiten zwischen den Eigenschaften der einzelnen Samen einer Samenmenge bestehen.

Zur Verbesserung der Handhabung und Messumgebung ist es bevorzugt, dass der Probenraum durch ein Probenbehältnis gebildet wird, dessen den Probenraum begrenzender Innenraum ein Volumen aufweist, welches auf die damit zu verwendende Saatgutart derart angepasst ist, dass eine Konzentration der während der Keimung beeinflussten (d.h. gebildeten und/oder verbrauchten) Zielkomponenten in der anfänglich in dem Probenraum vorhandenen Gaszusammensetzung in einem durch die Anzahl von elektrochemischen Gassensoren messbaren Bereich liegt.

Es ist möglich, mit dem erfindungsgemäßen Gasmesssystem mehr als einen Qualitätsparameter zu bestimmen und bereitzustellen. So können beispielweise zwei Qualitätsparameter bestimmt und bereitgestellt werden. Die Bereitstellung lediglich eines Qualitätsparameters ist jedoch bevorzugt. Es ist auch möglich, eine Mehrzahl von Qualitätsparametern (z.B. gewichtet) miteinander zu kombinieren, um einen kombinierten Qualitätsparameter als Qualitätsparameter bereitzustellen.

Das erfindungsgemäße Gasmesssystem kann als kompakte Baugruppe ausgestaltet sein oder örtlich verteilt (z.B. als Multi-Sensor-Array) ausgestaltet sein.

Beispielsweise kann die Anzahl von elektrochemischen Gassensoren an dem Probenraum angeordnet und mit diesem unmittelbar strömungstechnisch verbunden sein. Alternativ hierzu kann die Anzahl von elektrochemischen Gassensoren von dem Probenraum örtlich getrennt angeordnet und mit diesem mittelbar über eine Leitung strömungstechnisch verbunden sein.

Die Auswerteeinheit kann beispielsweise als eine elektrische oder elektronische Schaltung und/oder als ein Mikroprozessor auf einer Platine ausgestaltet sein. Wenn die Auswerteeinheit als Platine ausgestaltet ist, ist es bevorzugt, dass diese an einem oder allen der Anzahl von elektrochemischen Gassensoren aufgenommen ist.

Die Auswerteeinheit kann ergänzend oder alternativ beispielsweise als eine Datenverarbeitungsanlage, wie als PC oder als handgehaltenes Gerät mit geeigneter Programmierung ausgestaltet sein. Wenn die Auswerteeinheit als Datenverarbeitungsanlage ausgestaltet ist, ist es bevorzugt, dass diese örtliche von der Anzahl von elektrochemischen Gassensoren getrennt bereitgestellt und mit dieser kabellos oder kabelgebunden verbunden oder verbindbar ist.

Das Gasmesssystem kann (als Teil der Auswerteeinheit oder als separate Komponente des Gasmesssystems) einen Potentiostaten umfassen oder eine Schaltung oder Programmierung aufweisen, welche als Potentiostat fungiert.

Die Zielkomponente kann ein gasförmiger Bestandteil des in dem Probenraum vorhandenen bzw. während der Keimung gebildeten Gases sein.

Es ist möglich, als Anzahl von elektrochemischen Gassensoren genau einen elektrochemischen Gassensor oder aber eine Mehrzahl von elektrochemischen Gassensoren bereitzustellen.

Dabei ist die Anzahl von elektrochemischen Gassensoren insgesamt eingerichtet, sowohl Ethanol als auch Sauerstoff zu bestimmen.

Jeder der Anzahl von elektrochemischen Gassensoren oder nur ein Teil der Anzahl von elektrochemischen Gassensoren kann dazu eingerichtet sein, genau eine Zielkomponente zu messen oder eine Mehrzahl von Zielkomponenten, bevorzugt gleichzeitig, zu messen.

Dabei ist die Anzahl von elektrochemischen Gassensoren insgesamt eingerichtet, sowohl Ethanol als auch Sauerstoff zu bestimmen.

Jeder Probenraum kann mit genau einem elektrochemischen Gassensor oder mit einer Mehrzahl von elektrochemischen Gassensoren strömungstechnisch verbunden sein. Jeder der Anzahl von elektrochemischen Gassensoren kann als Einfachsensor oder als Mehrfachsensor, beispielsweise als Doppelsensor oder als Dreifachsensor ausgestaltet sein. Insofern ist es möglich, dass ein elektrochemischer Gassensor, einige elektrochemische Gassensoren oder alle elektrochemischen Gassensoren der Anzahl von elektrochemischen Gassensoren genau ein Messsignal bereitstellt bzw. bereitstellen, wenn dieser bzw. dieser als Einfachsensor ausgestaltet ist bzw. sind oder eine Mehrzahl von Messsignalen bereitstellt bzw. bereitstellen, wenn dieser bzw. diese als Mehrfachsensor ausgestaltet ist bzw. sind.

Es ist bevorzugt, dass jeder Probenraum mit einer Mehrzahl von Einfachsensoren und/ oder mit zumindest einem Mehrfachsensor strömungstechnisch verbunden ist, wobei die Mehrzahl von Einfachsensoren und/oder der Mehrfachsensor eingerichtet ist, als Zielkomponenten Sauerstoff und Ethanol zu messen.

Ferner erfindungsgemäß ist die Anzahl von elektrochemischen Gassensoren eingerichtet, eine Konzentration von Ethanol als eine Zielkomponente der Anzahl von Zielkomponenten zu bestimmen. Ergänzend ist die Anzahl von elektrochemischen Gassensoren eingerichtet, eine Konzentration von Sauerstoff als (weitere) Zielkomponente der Anzahl von Zielkomponenten zu bestimmen.

Es ist oben beschrieben worden, dass zumindest durch Betrachtung des zeitlichen Verlaufs einer Ethanolkonzentration des in dem Probenraum vorhandenen Gases oder einer aus diesem Wert bestimmbaren Ethanolmenge eine Aussage über die Keimfähigkeit des Saatguts gewonnen werden kann. Im Rahmen der Erfindung wurde ferner erkannt, dass eine ergänzende Bestimmung von Sauerstoff als Zielkomponente die Bestimmung des Qualitätsparameters noch besser an das zu untersuchende Verhalten des Saatguts angepasst werden.

So gehen die Erfinder, ohne an diese Theorie gebunden zu sein, davon aus, dass zwischen der zeitlichen Entwicklung einer Sauerstoffkonzentration und/oder einer Sauerstoffmenge und/oder einer Kohlenstoffdioxidkonzentration und/oder einer Kohlenstoffdioxidmenge und dem Keimungszustand des Samens eine Korrelation bestehen kann, welche in die Bestimmung des Qualitätsparameters einbezogen oder zur Bestimmung eines oder mehrerer zusätzlicher Qualitätsparameter berücksichtigt werden kann.

Vorzugsweise erfolgt die kontinuierliche Bestimmung der Anzahl von Konzentrationen und die Bereitstellung der Messsignale mit einem Takt von weniger als 10 Minuten, bevorzugt von weniger als 5 Minuten, weiter bevorzugt von weniger als 10 Sekunden, noch weiter bevorzugt von weniger als 5 Sekunden, ganz besonders bevorzugt von weniger als einer Sekunde.

Auf diese Weise kann die Abtastrate des Gasmesssystems an das dynamische Verhalten der Keimung des Samens angepasst und die zur Bestimmung des Qualitätsparameters relevanten Messsignale mit ausreichender zeitlicher Auflösung gewonnen werden.

Vorzugsweise indiziert der Qualitätsparameter eine Keimfähigkeit und vorzugsweise ferner eine Triebkraft des Saatguts.

Vorzugsweise weist das Gasmesssystem eine Mehrzahl von Probenräumen und eine entsprechende Mehrzahl von elektrochemischen Gassensoren auf, wobei jeweils ein Probenraum mit jeweils einem elektrochemischen Gassensor eine Untereinheit des Gasmesssystems bildet, so dass das Gasmesssystem eine Mehrzahl von Untereinheiten zur Aufnahme einer entsprechenden Mehrzahl von Samen des Saatguts umfasst.

In anderen Worten kann auf diese Art und Weise ein Multi-Sensor-Array bereitgestellt werden.

Auf diese Weise können vorteilhaft mehrere Einzelmessungen parallel durchgeführt werden, so dass eine Stichprobenmenge zur Bestimmung des Qualitätsparameters vergrößert werden kann.

Vorzugsweise weist das Gasmesssystem ferner eine Anzahl von Sensoren zur Überwachung einer Keimungsumgebung des Samens oder der Mehrzahl von Samen auf.

Die Anzahl von Sensoren kann als Einzahl oder Mehrzahl ausgestaltet sein.

Es ist bevorzugt, dass die Anzahl von Sensoren zur Überwachung einer Keimungsumgebung eingerichtet ist, Informationen über Bedingungen in dem Probenraum oder in den Probenräumen bereitzustellen, welche die Keimung des Samens oder der Samen beeinflussen. Hierbei kann es sich beispielsweise um eine Feuchte und/oder Temperatur in dem Probenraum oder in den Probenräumen handeln. Die Anzahl von Sensoren kann hierzu beispielsweise in dem Probenraum oder in den Probenräumen angeordnet sein. Jeder der Anzahl von Sensoren kann beispielsweise als ein Feuchtesensor und/oder als ein Temperatursensor ausgestaltet sein. Eine ergänzende oder alternativ visuelle Überwachung der Keimungsbedingungen ist ebenfalls möglich, wozu ein Sensor zur Überwachung der Keimungsumgebung beispielsweise als Kamera ausgestaltet sein kann.

Es ist bevorzugt, dass in jedem Probenraum ein Feuchtesensor und/oder ein Temperatursensor angeordnet und über eine oder die energietechnische und/oder datentechnische Schnittstelle mit einer oder der Auswerteeinheit verbunden ist.

Es ist möglich, in einem, einigen oder allen Probenräumen eine Mehrzahl gleichartiger vorbezeichneter Sensoren bereitzustellen, so dass ein Gradient der jeweils bestimmten Eigenschaft der Keimungsumgebung innerhalb

Diese und weitere Merkmale und Vorteile der Erfindung ergeben sich auch aus der nachstehenden Figurenbeschreibung. Dabei zeigt:
- **Fig. 1a**: eine schematische Darstellung eines erfindungsgemäßen Gasmesssystems,
- **Fig. 1b**: eine schematische Darstellung eines weiteren erfindungsgemäßen Gasmesssystems,
- **Fig. 2**: eine schematische Darstellung einer Variante des erfindungsgemäßen Gasmesssystems, und
- **Fig. 3**: eine schematische Darstellung von zwei Messsignalverläufen über die Zeit.

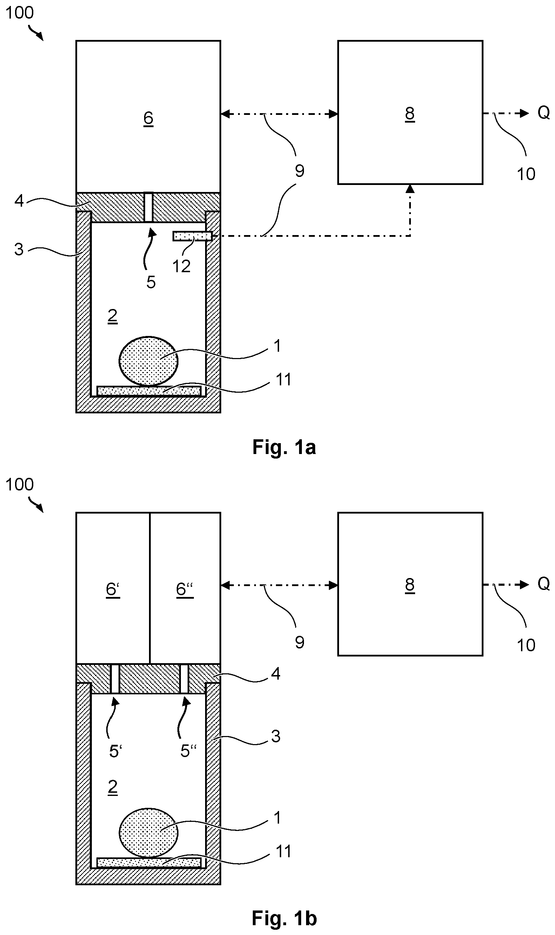

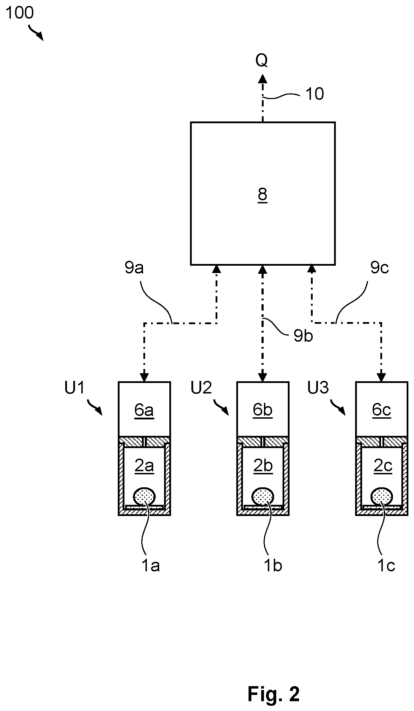

Erfindungsgemäß wird ein Gasmesssystem 100 bereitgestellt. Ein Beispiel eines solchen Gasmesssystems 100 ist in Fig. 1a dargestellt. Eine Variante eines erfindungsgemäßen Gasmesssystems 100 ist in Fig. 1b dargestellt. Eine weitere Variante eines erfindungsgemäßen Gasmesssystems 100 ist in Fig. 2 dargestellt.

Insofern im Folgenden allgemein von dem Gasmesssystem 100 gesprochen wird, sind alle Varianten erfindungsgemäßer Gasmesssysteme 100 gemeint.

Das Gasmesssystem 100 dient zur Bestimmung eines Qualitätsparameters Q von Saatgut.

Das Gasmesssystem 100 weist einen Probenraum 2, 2a, 2b, 2c zur Aufnahme eines Samens 1, 1a, 1b, 1c des Saatguts auf. Der Probenraum 2, 2a, 2b, 2c kann beispielsweise als im Wesentlichen zylindrisches oder quaderförmiges Probenbehältnis 3 ausgeführt sein. Der Probenraum 2, 2a, 2b, 2c kann neben dem Samen 1, 1a, 1b, 1c beispielsweise ferner ein Vlies 11 zur Feuchteregulierung des Probenraums 2, 2a, 2b, 2c aufnehmen. Der Probenraum 2, 2a, 2b, 2c kann beispielsweise durch einen Deckel 4 verschließbar sein. Deckel 4 und Probenbehältnis 3 können gegenüber der Umgebung abgedichtet sein oder eine vorbestimmte Gasdurchlässigkeit aufweisen. Wenn ein Deckel 4 bereitgestellt ist, ist es bevorzugt, dass in dem Deckel 4 eine Durchgangsöffnung 5 ausgebildet ist, wie dies in Fig. 1 und Fig. 2 ersichtlich ist. In dem Deckel 4 können auch mehrere Durchgangsöffnungen 5', 5" ausgebildet sein, wie dies in Fig. 2 ersichtlich ist.

Das Gasmesssystem 100 weist ferner eine Anzahl von elektrochemischen Gassensoren 6, 6', 6" 6a, 6b, 6c auf, welche strömungstechnisch mit dem Probenraum 2, 2a, 2b, 2c verbunden ist, um eine Anzahl von Konzentrationen einer Anzahl von Zielkomponenten eines in dem Probenraum 2, 2a, 2b, 2c vorhandenen Gases zu bestimmen und mit der Anzahl von Konzentrationen korrespondiere Messignale M, M1, M2, ... bereitzustellen.

Wenn ein Deckel 4 mit Durchgangsöffnung 5, 5', 5" bereitgestellt ist, es bevorzugt, dass mittels der Durchgangsöffnung 5, 5', 5" eine strömungstechnische Verbindung zwischen dem Probenraum 2, 2a, 2b, 2c und der Anzahl von elektrochemischen Gassensoren 6, 6', 6", 6a, 6b, 6c erreicht wird. In den Ausführungsbeispielen nach Fig. 1a und 2 ist hierzu ein elektrochemischer Gassensor 6 bzw. eine Mehrzahl von elektrochemischen Gassensoren 6a, 6b, 6c mittels einer Durchgangsöffnung 5 bzw. einer Mehrzahl von Durchgangsöffnungen 5a, 5b, 5c mit dem Probenraum 2 bzw. den Probenräumen 2a, 2b, 2c verbunden. In dem Ausführungsbeispiel nach Fig. 1b sind hierzu eine Mehrzahl von elektrochemischen Gassensoren 6', 6" mittels einer Mehrzahl von Durchgangsöffnungen 5', 5" mit dem Probenraum 2 verbunden.

Es ist bevorzugt, dass die Messsignale M, M1, M2, ... über eine energietechnische und/oder datentechnische Schnittstelle 9, 9a, 9b, 9c bereitgestellt werden.

Die Bestimmung der Anzahl von Konzentrationen und die Bereitstellung der Messsignale M, M1, M2 erfolgt erfindungsgemäß kontinuierlich.

Das erfindungsgemäße Gasmesssystem 100 weist ferner eine Auswerteeinheit 8 auf, welche eingerichtet ist: die Messsignale M, M1, M2, ... zum - Beispiel über die energietechnische und/oder datentechnische Schnittstelle 9, 9a, 9b, 9c - automatisch zu empfangen, den Qualitätsparameter Q aus den Messsignalen M, M1, M2, ... zu bestimmen, und den Qualitätsparameter Q über eine datentechnische Schnittstelle 10 bereitzustellen. Die datentechnische Schnittstelle 10 kann beispielsweise als Verbindung mit einem PC oder mit einem Display ausgestaltet sein.

Es ist in dem Ausführungsbeispiel nach Fig. 1a dargestellt, aber in allen Ausführungsbeispielen möglich, dass das Gasmesssystem 100 ferner eine Anzahl von Sensoren 12 zur Überwachung einer Keimungsumgebung des Samens 1 oder der Mehrzahl von Samen 1a, 1b, 1c aufweisen kann. Die Anzahl von Sensoren 12 kann dabei vorzugsweise über die energietechnische und/oder datentechnische Schnittstelle 9, 9a, 9b, 9c mit der Auswerteeinheit 8 verbunden sein. Die durch die Anzahl von Sensoren 12 gewonnen Informationen über die Keimungsumgebung und somit die Keimungsbedingungen des Samens 1 oder der Mehrzahl von Samen 1a, 1b, 1c kann in die Bestimmung des Qualitätsparameter Q einbezogen werden.

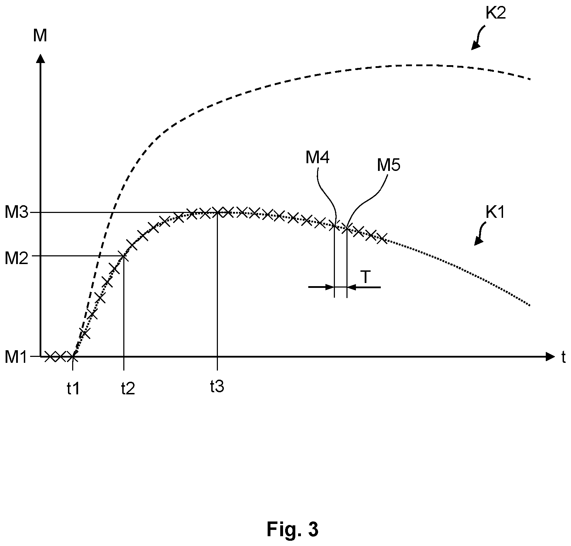

In Fig. 3 ist eine schematische Darstellung von zwei Messkurven K1, K2 dargestellt, welche sich mit dem erfindungsgemäßen Gasmesssystem 100 ermitteln lassen.

Messkurve K1 zeigt dabei den Verlauf der Messsignale M, M1, M2, ... über die Zeit t, wobei jedes der kontinuierlich aufgenommenen Messsignale M, M1, M2, ... bereitzustellen als Kreuze angedeutet sind. Durch Schritte der Datenverarbeitung, beispielsweise durch Interpolation, kann aus den Messsignalen M, M1, M2, ... eine Messkurve K1, K2 erhalten werden. Messkurve K1 zeigt dabei ein Messignal M- Zeit t-Verhalten eines guten Samens1, 1a, 1b, 1c, während Messkurve K2 ein Messignal M-Zeit t-Verhalten eines schlechten Samens 1, 1a, 1b, 1c zeigt.

Es ist ersichtlich, dass durch den qualitativen Verlauf der jeweiligen Messkurven K1, K2 und durch quantitative Parameter der jeweiligen Messkurven K1, K2 charakteristischen Kenngrößen des untersuchten Samens 1, 1a, 1b, 1c erhalten werden können, welche ein Qualitätsparameter des entsprechenden Saatguts indizieren. Beispielsweise kann aus den Messignalen M, M1, M2, ... direkt oder aus der aus diesen erhältlichen Messkurve K1, K2 ein Zeitpunkt t1 ermittelt werden, zu welchem eine Keimung beginnt, ein Zeitpunkt t2 ermittelt werden, zu welchem in dem Messignal M- Zeit t-Verlauf ein Wendepunkt vorliegt und ein Zeitpunkt t3 ermittelt werden, zu welchem ein Messsignal M-Maximum vorliegt. Ferner beispielsweise ist es möglich, die erhaltenen Messkurven K1, K2 zu integrieren, um eine Aussage über einer während der Messung gebildeten Gesamtmenge der Anzahl von Zielkomponenten zu erhalten. Abhängig davon, welche Eigenschaft der Keimung des Samens zur Beurteilung der Qualität herangezogen werden soll, kann aus allen oder einigen dieser charakteristischen Parameter der Qualitätsparameter Q bestimmt werden.

Es ist bevorzugt, dass der Qualitätsparameter Q eine Keimfähigkeit und vorzugsweise ferner eine Triebkraft des Saatguts indiziert.

Es ist erfindungsgemäß, dass die Anzahl von elektrochemischen Gassensoren 6, 6', 6", 6a, 6b, 6c eingerichtet ist, eine Konzentration von Ethanol Et als eine Zielkomponente der Anzahl von Zielkomponenten zu bestimmen. Ergänzend ist es erfindungsgemäß, dass die Anzahl von elektrochemischen Gassensoren 6, 6', 6", 6a, 6b, 6c eingerichtet ist, eine Konzentration von Sauerstoff als Zielkomponente der Anzahl von Zielkomponenten zu bestimmen.

Es ist ferner bevorzugt, dass die kontinuierliche Bestimmung der Anzahl von Konzentrationen und die Bereitstellung der Messsignale M, M1, M2, ... mit einem Takt T von weniger als 10 Minuten, bevorzugt weniger als 5 Minuten, weiter bevorzugt von weniger als 10 Sekunden, noch weiter bevorzugt weniger als 5 Sekunden, ganz besonders bevorzugt von weniger als einer Sekunde erfolgt. In Fig. 3 ist insofern der zeitliche Abstand und somit der Takt T, in welchem die Messsignale M4 und M5 erhalten werden, schematisch angedeutet. Es ist bevorzugt, dass der Takt T, welcher zwischen zwei aufeinanderfolgenden Messsignalen M, M1, M2, ... liegt, konstant ist. Das Gasmesssystem 100 nach Fig. 2 unterscheidet sich von den Gasmesssystemen nach Fig. 1a und 1b dadurch, dass eine Mehrzahl von vorbeschriebenen Probenräumen 2a, 2b, 2c und eine entsprechende Mehrzahl von elektrochemischen Gassensoren 6a, 6b, 6c vorgesehen ist, wobei jeweils ein Probenraum 2a, 2b, 2c mit jeweils einem elektrochemischen Gassensor 6a, 6b, 6c eine Untereinheit U1, U2, U3 des Gasmesssystems 100 bildet, so dass das Gasmesssystem 100 eine Mehrzahl von Untereinheiten U1, U2, U3 zur Aufnahme einer entsprechenden Mehrzahl von Samen 1a, 1b, 1c des Saatguts umfasst. Es wird somit ein Multi-Sensor-Array-Gasmesssystem 100 gebildet. Auch in dieser Variante kann jeder Deckel 4 eine Mehrzahl von Durchgangsöffnungen 5', 5"aufweisen, um mit einer Mehrzahl von elektrochemischen Gassensoren 6', 6" oder einem Mehrfachsensor in strömungstechnischer Verbindung zu stehen.

Während in Fig. 2 lediglich drei Probenräume 2a, 2b, 2c und drei elektrochemischen Gassensoren 6a, 6b, 6c dargestellt sind, ist die Mehrzahl von Probenräumen 2a, 2b, 2c und die entsprechende Mehrzahl von elektrochemischen Gassensoren 6a, 6b, 6c beliebig skalierbar.

### Bezugszeichenliste

100 Gasmesssystem
1, 1a, 1b, 1c Samen
2, 2a, 2b, 2c Probenraum
3 Probenbehältnis
4 Deckel
5, 5', 5" Durchgangsöffnung
6, 6', 6" 6a, 6b, 6c elektrochemischer Gassensor
7 Messsignale
8 Auswerteeinheit
9, 9a, 9b, 9c energietechnische und/oder datentechnische Schnittstelle
10 datentechnische Schnittstelle
11 Vlies
12 Sensor
Q Qualitätsparameter
Et Ethanol
K1, K2 Messkurve
M, M1, M2, ... Messsignal
t1, t2, t3 Zeit
T Takt
U1, U2, U3 Untereinheit

## Patentansprüche

1. Gasmesssystem (100) zur Bestimmung eines Qualitätsparameters (Q) von Saatgut, aufweisend:
- einen Probenraum (2, 2a, 2b, 2c) zur Aufnahme eines Samens (1, 1a, 1b, 1c) des Saatguts,
- eine Anzahl von elektrochemischen Gassensoren (6, 6', 6", 6a, 6b, 6c), welche strömungstechnisch mit dem Probenraum (2, 2a, 2b, 2c) verbunden ist, um eine Anzahl von Konzentrationen einer Anzahl von Zielkomponenten eines in dem Probenraum (2, 2a, 2b, 2c) vorhandenen Gases zu bestimmen und mit der Anzahl von Konzentrationen korrespondierende Messignale (M, M1, M2) bereitzustellen,
wobei die Anzahl von elektrochemischen Gassensoren (6, 6', 6", 6a, 6b, 6c) eingerichtet ist, eine Konzentration von Ethanol (Et) als eine Zielkomponente der Anzahl von Zielkomponenten zu bestimmen, und
**dadurch gekennzeichnet, dass**
die Anzahl von elektrochemischen Gassensoren (6, 6', 6", 6a, 6b, 6c) eingerichtet ist, eine Konzentration von Sauerstoff als eine weitere Zielkomponente der Anzahl von Zielkomponenten zu bestimmen,
wobei die Bestimmung der Anzahl von Konzentrationen und die Bereitstellung der Messsignale (M, M1, M2) kontinuierlich erfolgt, und
- eine Auswerteeinheit (8), welche eingerichtet ist:
- die Messsignale (M, M1, M2) automatisch zu empfangen,
- den Qualitätsparameter (Q) aus den Messsignalen (M, M1, M2) zu bestimmen, und
- den Qualitätsparameter (Q) über eine datentechnische Schnittstelle (10) bereitzustellen.

2. Gasmesssystem (100) nach Anspruch 1,
wobei die kontinuierliche Bestimmung der Anzahl von Konzentrationen und die Bereitstellung der Messsignale (M, M1, M2) mit einem Takt (T) von weniger als 10 Minuten, bevorzugt weniger als 5 Minuten, weiter bevorzugt von weniger als 10 Sekunden, noch weiter bevorzugt weniger als 5 Sekunden, ganz besonders bevorzugt von weniger als einer Sekunde erfolgt.

3. Gasmesssystem (100) nach einem der vorherigen Ansprüche,
wobei der Qualitätsparameter (Q) eine Keimfähigkeit und vorzugsweise ferner eine Triebkraft des Saatguts indiziert.

4. Gasmesssystem (100) nach einem der vorherigen Ansprüche,
aufweisend eine Mehrzahl von Probenräumen (2a, 2b, 2c) und eine entsprechende Mehrzahl von elektrochemischen Gassensoren (6a, 6b, 6c),
wobei jeweils ein Probenraum (2a, 2b, 2c) mit jeweils einem elektrochemischen Gassensor (6a, 6b, 6c) eine Untereinheit (U1, U2, U3) des Gasmesssystems (100) bildet, so dass das Gasmesssystem (100) eine Mehrzahl von Untereinheiten (U1, U2, U3) zur Aufnahme einer entsprechenden Mehrzahl von Samen (1a, 1b, 1c) des Saatguts umfasst.

5. Gasmesssystem (100) nach einem der vorherigen Ansprüche,
ferner aufweisend eine Anzahl von Sensoren (12) zur Überwachung einer Keimungsumgebung des Samens (1) oder der Mehrzahl von Samen (1a, 1b, 1c).

## Claims

1. Gas measuring system (100) for determining a quality parameter (Q) of seed stock, having:
- a sample chamber (2, 2a, 2b, 2c) for receiving a seed (1, 1a, 1b, 1c) of the seed stock,
- a number of electrochemical gas sensors (6, 6', 6'', 6a, 6b, 6c) which are fluidically connected to the sample chamber (2, 2a, 2b, 2c) in order to determine a number of concentrations of a number of target components of a gas present in the sample chamber (2, 2a, 2b, 2c) and to provide measurement signals (M, M1, M2) corresponding to the number of concentrations,
wherein the number of electrochemical gas sensors (6, 6', 6'', 6a, 6b, 6c) is configured to determine a concentration of ethanol (Et) as a target component of the number of target components, and
**characterized in that**
the number of electrochemical gas sensors (6, 6', 6'', 6a, 6b, 6c) is configured to determine a concentration of oxygen as a further target component of the number of target components,
wherein the determination of the number of concentrations and the provision of the measurement signals (M, M1, M2) take place continuously, and
- an evaluation unit (8) which is configured:
- to receive the measurement signals (M, M1, M2) automatically,
- to determine the quality parameter (Q) from the measurement signals (M, M1, M2), and
- to provide the quality parameter (Q) via a data interface (10).

2. Gas measuring device (100) according to claim 1,
wherein the continuous determination of the number of concentrations and the provision of the measurement signals (M, M1, M2) take place in a cycle time (T) of less than 10 minutes, preferably less than 5 minutes, more preferably less than 10 seconds, even more preferably less than 5 seconds, most preferably less than one second.

3. Gas measuring system (100) according to any of the preceding claims,
wherein the quality parameter (Q) indicates a germination capacity and preferably also a sprouting force of the seed stock.

4. Gas measuring system (100) according to any of the preceding claims,
having a plurality of sample chambers (2a, 2b, 2c) and a corresponding plurality of electrochemical gas sensors (6a, 6b, 6c),
wherein each sample chamber (2a, 2b, 2c) with a respective electrochemical gas sensor (6a, 6b, 6c) forms a subunit (U1, U2, U3) of the gas measuring system (100) so that the gas measuring system (100) comprises a plurality of subunits (U1, U2, U3) for receiving a corresponding plurality of seeds (1a, 1b, 1c) of the seed stock.

5. Gas measuring system (100) according to any of the preceding claims,
further having a number of sensors (12) for monitoring a germination environment of the seed (1) or of the plurality of seeds (1a, 1b, 1c).

## Revendications

1. Système de mesure de gaz (100) permettant de déterminer un paramètre de qualité (Q) de semences, présentant :
- une chambre d'échantillonnage (2, 2a, 2b, 2c) permettant de recevoir une graine (1, 1a, 1b, 1c) des semences,
- un certain nombre de capteurs de gaz électrochimiques (6, 6', 6", 6a, 6b, 6c) qui sont reliés par communication fluidique à la chambre d'échantillonnage (2, 2a, 2b, 2c) afin de déterminer un nombre de concentrations d'un nombre de composants cibles d'un gaz présent dans la chambre d'échantillonnage (2, 2a, 2b, 2c) et de fournir des signaux de mesure (M, M1, M2) correspondant au nombre de concentrations,
dans lequel le nombre de capteurs de gaz électrochimiques (6, 6', 6", 6a, 6b, 6c) est configuré pour déterminer une concentration d'éthanol (Et) en tant que composant cible parmi le nombre de composants cibles, et
**caractérisé en ce que**
le nombre de capteurs de gaz électrochimiques (6, 6', 6", 6a, 6b, 6c) est configuré pour déterminer une concentration d'oxygène en tant qu'autre composant cible parmi le nombre de composants cibles,
dans lequel la détermination du nombre de concentrations et la fourniture des signaux de mesure (M, M1, M2) sont effectuées en continu, et
- une unité d'analyse (8) qui est configurée pour :
- recevoir automatiquement les signaux de mesure (M, M1, M2),
- déterminer le paramètre de qualité (Q) à partir des signaux de mesure (M, M1, M2), et
- fournir le paramètre de qualité (Q) par l'intermédiaire d'une interface informatique (10).

2. Système de mesure de gaz (100) selon la revendication 1,
dans lequel la détermination en continu du nombre de concentrations et la fourniture des signaux de mesure (M, M1, M2) sont effectuées à une cadence (T) inférieure à 10 minutes, de préférence inférieure à 5 minutes, plus préférablement inférieure à 10 secondes, encore plus préférablement inférieure à 5 secondes, de manière tout particulièrement préférée inférieure à une seconde.

3. Système de mesure de gaz (100) selon l'une des revendications précédentes,
dans lequel le paramètre de qualité (Q) indique la capacité de germination et, de préférence en outre, la force de croissance des semences.

4. Système de mesure de gaz (100) selon l'une des revendications précédentes,
présentant une pluralité de chambres d'échantillonnage (2a, 2b, 2c) et une pluralité correspondante de capteurs de gaz électrochimiques (6a, 6b, 6c),
dans lequel respectivement une chambre d'échantillonnage (2a, 2b, 2c) comportant respectivement un capteur de gaz électrochimique (6a, 6b, 6c) forme une sous-unité (U1, U2, U3) du système de mesure de gaz (100), de sorte que le système de mesure de gaz (100) comprend une pluralité de sous-unités (U1, U2, U3) permettant de recevoir une pluralité correspondante de graines (1a, 1b, 1c) des semences.

5. Système de mesure de gaz (100) selon l'une des revendications précédentes,
présentant en outre un certain nombre de capteurs (12) permettant de surveiller l'environnement de germination de la graine (1) ou de la pluralité de graines (1a, 1b, 1c).
